# EUROPEAN PATENT APPLICATION

(11) **EP 4 293 685 A1**
(43) Date of publication of application: **20.12.2023**
(21) Application number: 22771711.3
(22) Date of filing: 14.03.2022
(51) Int. Cl.: G16H 50/50, G16H 50/70, G16H 50/20, G06N 3/08

(54) **PROGNOSIS PREDICTION METHOD USING RESULT OF DISEASE DIAGNOSIS THROUGH NEURAL NETWORK AND SYSTEM THEREFOR**

(30) Priority: 16.03.2021 KR 20210034257; 21.10.2021 KR 20210141101
(71) Applicant: Deep Bio Inc., Seoul 08380 (KR)
(72) Inventor: KIM, Sun Woo, Seongnam-si, Gyeonggi-do 13599 (KR)
(74) Representative: Flügel Preissner Schober Seidel
(86) International application number: PCT/KR2022/003547
(87) International publication number: WO 2022/197045

(57) **Abstract**

Disclosed are a prognosis prediction method using a result of disease diagnosis through a neural network and a system therefor. The prognosis prediction method comprises: a step in which the system receives a biometric image as an input; a step in which the system generates an expression region diagnosis result in which an expression region in the biometric image, in which a disease has been expressed, is determined with respect to the input biometric image; and a step in which the system determines first information corresponding to the size of the entire tissue in the biometric image and second information corresponding to the size of the expression region in the biometric image, on the basis of the diagnosis result, and generates prognosis prediction information based on a result of the determination, wherein the step in which the system generates the expression region diagnosis result in which the expression region in the biometric image, in which the disease has been expressed, is determined with respect to the input biometric image comprises generating the expression region diagnosis result on the basis of a result of determining whether the disease has been expressed for each of certain patches in which the biometric image is divided into certain sizes, or generating the expression region diagnosis result on the basis of a result of segmenting, for each of the patches, a region in a patch, in which the disease has been expressed.

## Description

### Technical Field

The present disclosure relates to a prognosis prediction method using a result of disease diagnosis through a neural network and a system therefor. More specifically, the present disclosure relates to a method and system capable of deriving a predictive factor for prognosis prediction of a disease using a diagnosis result of a disease diagnosis system that is capable of segmenting a development region of a disease through a neural network and performing prognosis prediction using the same.

### Background Art

One of main tasks performed by the pathology or pathology department is to perform diagnosis to determine the condition or symptom of a specific disease by reading a patient's biometric image. This diagnosis is a method that relies on the experience and knowledge of skilled medical personnel for a long time.

Recently, thanks to the development of machine learning, attempts to automate tasks such as recognizing or classifying images by computer systems have been actively made. In particular, attempts have been made to automate the diagnosis that has been performed by skilled medical personnel using a neural network (e.g., a deep learning method using a convolution neural network (CNN)), which is a type of machine learning.

In particular, diagnosis through deep learning using the neural network (e.g., CNN) does not simply automate the experience and knowledge of conventional skilled medical personnel, but in that it finds the characteristic elements through self-learning and derives the desired answer, in some cases, the characteristics of disease factors that the skilled medical personnel were not aware of may be found in images.

In general, the diagnosis of diseases through the neural network using biometric images involves the use of fragments of biometric images, that are, patches (also referred to as tiles). In other words, for the tile, the skilled medical professional annotates a state of a specific disease (e.g., whether cancer is developed) and trains the neural network using a plurality of these annotated tiles as training data. In this case, the convolution neural network may be used for the neural network.

On the other hand, for prognosis prediction of the disease, information on how large the development site of a disease occupies in the entire tissue to be diagnosed, that is, a development rate, may be used as a crucial factor. Since the disease progression is predicted by these development rate while appropriate treatment methods, surgery, and drug selection are carried out, the development rate may be very important information for prognosis prediction.

However, in the past, pathologists visually observed a glass slide in which a tissue specimen is included using a microscope and marked the development region determined by oneself on the glass slide with a predetermined tool (e.g., a marker), only resulting in derivation of a rough development rate based on the marked information.

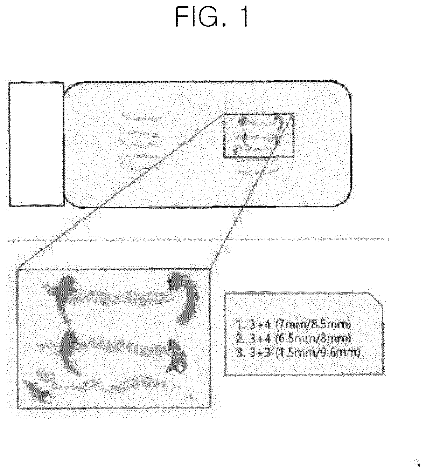

The determination method for the conventional development rate is shown in FIG. 1.

FIG. 1 is a diagram for explaining a method in which the development rate of a disease is determined by a conventional pathologist.

Referring to FIG. 1, previously, as shown in an upper part of FIG. 1, at least one biotissue is stained in a predetermined manner and then placed on a slide.

Then, as shown at a lower part of FIG. 1, the pathologist visually checks the tissues placed on these slides one by one under the microscope and marks the region having the disease developed in each tissue. In this case, since the marking is made on the development region of the disease while visually checking the region, it is practically impossible to mark in very fine unit of region (for example, gland units in the case of prostate cancer), and as shown in the lower part of FIG. 1, only the minimum information to determine the approximate development rate is marked such as a method of making an end point in one direction of the development region of the disease and an end point in the other direction.

Shown in the lower part of FIG. 1 is an example in which the pathologist marked directly on the slide (glass slide) with a red writing tool. In addition, although FIG. 1 illustrates an exemplary case in which the disease is prostate cancer, an average expert in the art of the present disclosure will be able to easily infer that the scope of the present disclosure is not limited thereto, and the technical spirit of the present disclosure may be applied to various diseases.

Then, in the case of the lower part of FIG. 1, information related to the development ratio that may be known by the pathologist for a first tissue may be information that a total length of the tissue is 8.5 mm and a length of the development region determined based on the marked information is 7 mm, while both Gleason scores 3 and 4 coexist in the developed region.

In addition, information related to the development rate that may be known by determination of the pathologist for a second tissue may be information that a total length of the tissue is 8 mm and a length of the developed region determined based on the marked information is 6.5 mm, while both Gleason scores 3 and 4 coexist in the developed region.

In addition, information related to the development rate that may be known by determination of the pathologist for a third tissue may be information that a total length of the tissue is 9.6 mm and a length of the developed region determined based on the marked information is 1.5mm, while there is only Gleason score of 3 in the developed region.

As such, when acquiring information related to the development rate for prognosis prediction based on determination of the pathologist, it is inevitable to be affected by the method of marking the development region of the disease, a thickness of the writing tool, and the physical condition of the pathologist at the time of the determination, probably bearing high inaccuracy.

In addition, as shown in FIG. 1, it is common for the pathologist to mark only the boundaries (e.g., both end points) of the development region of the disease and predict the development rate with assumption that the disease is developed over the entire region within the boundaries. However, in reality, it is difficult to determine an exact development rate due to coexistence of regions with and without the disease developed within the boundary.

In addition, as described in FIG. 1, when the disease progression (e.g., Gleason score for prostate cancer) is distinguished even if the disease is developed, it is practically difficult to acquire such information even if the development rate for each disease progression state may become important information (e.g., the development rate only for Gleason score of 3 and the development rate only for Gleason score of 4 in the entire tissue).

Therefore, there is an urgent need for a method of accurately obtaining information for prognosis prediction using diagnosis results with neural networks to perform prognosis prediction of a diseases and a system therefor.

### [Prior Art Document]

### - Patent Document

(Patent Document1) Korean Patent Application Publication No. 10-2019-0084814 "Disease diagnosis system and method for performing segmentation using neural network and non-local block"

### Disclosure of the Invention

### Technical Goals

A technical object to be achieved by the present disclosure is to provide a technical idea for accurately obtaining information for prognosis prediction using a diagnosis result using a neural network and performing prognosis prediction of a disease using the same.

In particular, an object of the present disclosure is to provide a technical idea for obtaining more accurate prognosis prediction information using a diagnosis result derived by performing segmentation for classification of a region having a disease developed even within a patch rather than determining that the disease is developed in a patch unit.

### Technical Solutions

According to one aspect of the present disclosure, a prognosis prediction method implemented by a system including a processor and a storage device configured to store a neural network includes receiving, by the system, a biometric image; generating for the received biometric image, by the system, a development region diagnosis result in which a development region having a disease developed is determined in the biometric image; and determining, by the system, first information corresponding to a size of an entire tissue on the biometric image and second information corresponding to a size of the development region on the biometric image based on the diagnosis result and generating prognosis prediction information based on a result of the determination, wherein the generating for the received biometric image, by the system, of the development region diagnosis result in which the development region having the disease developed is determined in the biometric image includes generating the development region diagnosis result based on a result of determining whether the disease is developed for each of predetermined patches obtained by dividing the biometric image into a predetermined size or generating the development region diagnosis result based on a result of segmentation of a region having the disease developed among the patches for each of the patches.

The generating for the received biometric image, by the system, of the development region diagnosis result in which the development region having the disease developed is determined in the biometric image may include generating, when the disease is developed and a state of the developed disease is classified into a plurality of classes, by the system, the development region diagnosis result for each class, wherein the determining, by the system, of the first information corresponding to the size of the entire tissue on the biometric image and the second information corresponding to the size of the development region on the biometric image based on the diagnosis result and generating prognosis prediction information based on the result of the determination may include determining, by the system, the second information corresponding to the size of the development region on the biometric image for each class based on the development region diagnosis result.

The prognosis prediction information may include information on a development rate corresponding to the size of the development region relative to the size of the entire tissue.

A system for prognosis prediction according to another aspect includes a processor and a memory configured to store a program driven by the processor and a neural network, wherein the processor is configured to drive the program to receive a biometric image, generate a development region diagnosis result in which a development region having a disease developed is determined in the biometric image for the biometric image, determine first information corresponding to a size of an entire tissue on the biometric image and second information corresponding to a size of a development region on the biometric image based on the diagnosis result, and generate prognosis prediction information based on a result of the determination, wherein the processor is configured to drive the program to generate the development region diagnosis result based on a result of determining whether the disease is developed for each of predetermined patches obtained by dividing the biometric image into a predetermined size or generate the development region diagnosis result based on a result of segmentation of a region having the disease developed among the patches for each of the patches.

The processor may be configured to drive the program to generate, when the disease is developed and a state of the developed disease is classified into a plurality of classes, the development region diagnosis result for each class and determine the second information corresponding to the size of the development region on the biometric image for each class based on the development region diagnosis result.

The above method may be implemented by a program installed in a data processing device.

### Advantageous Effects

According to the technical idea of the present disclosure, it is possible to obtain prognosis prediction information that enables relatively accurate prognosis prediction with a diagnosis result using a neural network to perform prognosis prediction of a disease using the same, thereby predicting prognosis based on accurate and objective information.

In addition, using diagnosis results derived by performing segmentation for classification of regions having a disease developed even in a patch rather than determining that the disease is developed in a patch unit, it is possible to obtain more precise prognosis prediction information.

### Brief Description of Drawings

In order to more fully understand the drawings cited in the detailed description of the present disclosure, a brief description of each drawing is provided.
FIG. 1 is a diagram for explaining a method in which a development rate of a disease is determined by a conventional pathologist.
FIG. 2 is a diagram illustrating a schematic system configuration for implementing a prognosis prediction method using a result of disease diagnosis through a neural network in accordance with the technical idea of the present disclosure.
FIG. 3 is a diagram for explaining a hardware configuration of a system in accordance with an embodiment of the present disclosure.
FIG. 4 is a diagram for explaining a logical configuration of a system in accordance with an embodiment of the present disclosure.
FIG. 5 is a diagram for illustrating an example that a system in accordance with an embodiment of the present disclosure is classified into a diagnosis system and a prognosis prediction system.
FIG. 6 is a diagram illustrating a development region diagnosis result of a biometric image to describe a prognosis prediction method using a result of disease diagnosis through a neural network in accordance with an embodiment of the present disclosure.
FIG. 7 is a diagram for explaining an entire structure of a patch level segmentation neural network in accordance with an embodiment of the present disclosure.

### Best Mode for Carrying Out the Invention

Since the present disclosure may be variously modified and have various embodiments, specific embodiments will be illustrated in the drawings and described in detail in the detailed description. However, this is not intended to limit the present disclosure to specific embodiments, and it should be understood to include all transformations, equivalents and substitutes included in the spirit and scope of the present disclosure. In describing the present disclosure, if it is determined that a detailed description of related known technologies may obscure the gist of the present disclosure, the detailed description will be omitted.

Terms such as first and second may be used to describe various components, but the components should not be limited by the terms. The terms are used only for the purpose of distinguishing one component from another.

The terms used in the present application are used only to describe a particular embodiment and are not intended to limit the present disclosure. Singular expressions include plural expressions unless the context clearly dictates otherwise.

In this specification, terms such as "include" or "have" are intended to designate the presence of features, numbers, steps, operations, components, parts, or combinations thereof described in the specification, and it should be understood that it does not preclude the possibility of the presence or addition of one or more other features, numbers, steps, operations, components, parts, or combinations thereof.

In addition, in the present specification, when one component 'transmits' data to another component, it means that the component may directly transmit the data to the other component, and that the data may be transmitted to the other component through at least one other component. Conversely, when one component 'directly transmits' data to another component, it means that the data is transmitted from the component to the other component without going through the other component.

Hereinafter, with reference to the accompanying drawings, the present disclosure will be described in detail based on embodiments of the present disclosure. Like reference numerals presented in each drawing indicate like members.

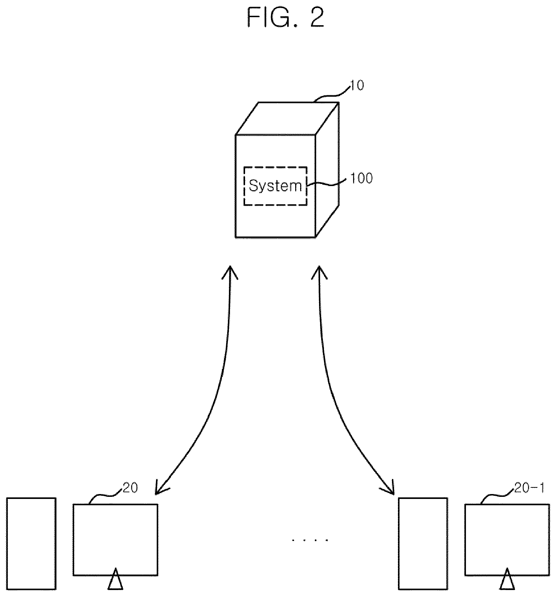

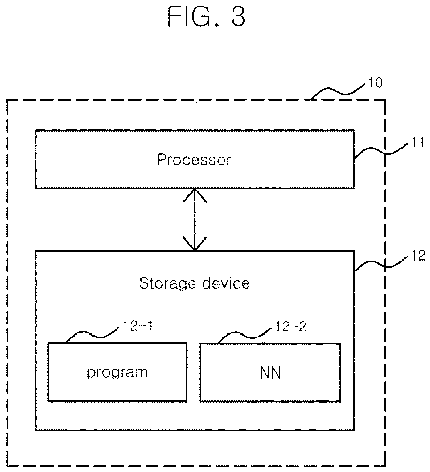

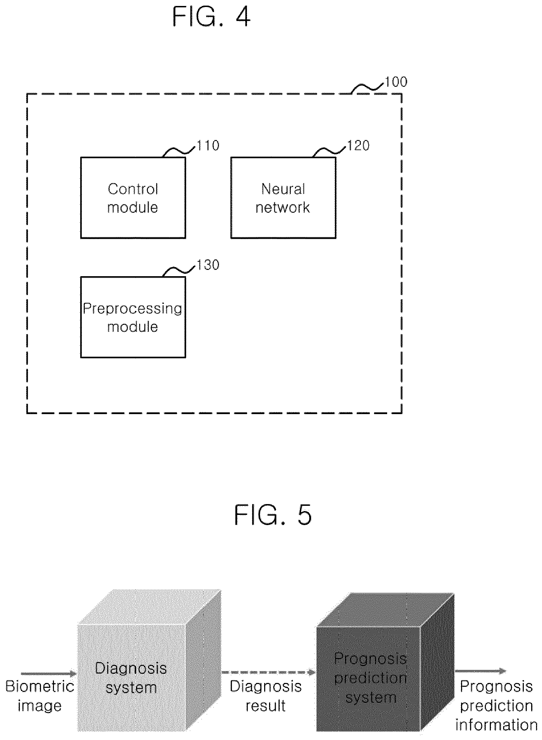

FIG. 2 is a diagram illustrating a schematic system configuration for implementing a prognosis prediction method using a result of disease diagnosis through a neural network in accordance with the technical idea of the present disclosure. FIG. 3 is a diagram for explaining a hardware configuration of a system in accordance with an embodiment of the present disclosure. FIG. 4 is a diagram for explaining a logical configuration of the system in accordance with an embodiment of the present disclosure. FIG. 5 is a diagram for illustrating an example that a system in accordance with an embodiment of the present disclosure is classified into a diagnosis system and a prognosis prediction system.

Referring to FIGS. 2 to 5, a system 100 for prognosis prediction using a result of disease diagnosis through a neural network may be provided to implement the technical idea of the present disclosure.

The system 100 according to the technical idea of the present disclosure may be installed on a predetermined server 10 to implement the technical idea of the present disclosure. An average expert in the art of the present disclosure will be able to easily infer that the server 10 refers to a data processing device having a computing ability for implementing the technical ideas of the present disclosure, and in general, any device capable of performing specific services, such as a personal computer and a mobile terminal, as well as a data processing device that is accessible to a client through a network, may be defined as a server.

The server 10 may include a processor 11 and a storage device (memory 12) as shown in FIG. 3. The processor 11 may refer to a computing device capable of driving a program 12-1 for implementing the technical idea of the present disclosure, and the processor 11 may perform diagnosis using the program 12-1 and a neural network 12-2 defined by the technical idea of the present disclosure.

The neural network 12-2 may determine a region in which a predetermined disease is developed in the biometric image. According to an example, the neural network 12-2 may have been trained to determine whether a disease is developed in a patch which is obtained by dividing the biometric image in a predetermined size. Such the neural network 12-2 may be defined as a patch level classification neural network that performs patch level diagnosis as described below.

According to an embodiment, the neural network 12-2 may include not only a patch level classification neural network, but also a neural network for classifying and specifying only the region in which the disease is developed among each of the patches, that is, a patch level segmentation neural network. The patch level segmentation neural network may perform patch level segmentation to specify a region in which a disease exists in the patch.

According to an embodiment, the neural network 12-2 may further include a neural network performing slide level diagnosis. According to an embodiment, the configuration for performing the slide level diagnosis may be implemented through various machine learning techniques as well as the neural network. According to the technical idea of the present disclosure, for a diagnosis engine for performing the slide level diagnosis, known XGBoost has been used, but it is apparent that diagnosis engines according to various machine learning techniques may be implemented, and such the diagnosis engine may be stored in the storage device 12.

The server 10 may include a processor 11 and a storage device 12 as shown in FIG. 3. The processor 11 may refer to a computing device capable of driving a program 12-1 for implementing the technical idea of the present disclosure, and the processor 11 may perform diagnosis using the program 12-1 and the neural network 12-2 defined by the technical idea of the present disclosure.

The neural network 12-2 may include a neural network that performs patch level diagnosis as described below. A neural network that performs patch level diagnosis may determine whether a disease exists in a patch that is a part of a segmented slide. In addition, a neural network performing patch level diagnosis may perform segmentation to specify a development region of a disease among the patches, and such the neural network will hereinafter be referred to as a patch level segmentation neural network.

The storage device 12 may refer to a data storage means capable of storing the program 12-1, the neural network 12-2, and/or a diagnosis engine performing slide level diagnosis and may be implemented as a plurality of storage means according to an embodiment. In addition, the storage device 12 may refer to inclusion of not only a main memory device included in the server 10 but also a temporary storage device or memory that may be included in the processor 11.

Although the system 100 is shown in FIG. 2 or FIG. 3 as being implemented as any one physical device, an average expert in the art of the present disclosure may easily infer that a plurality of physical devices may be organically combined as needed to implement the system 100 according to the technical idea of the present disclosure.

In the present specification, when the system 100 performs diagnosis, it may refer to a series of processes of receiving a biometric image in which the biotissue is expressed, that is, a whole slide or a patch that is a part of the slide to output output data that is defined in this specification.

According to an example, the system 100 may perform two phase diagnosis. A first phase may be a process of performing patch level diagnosis, and in this process, the system 100 may receive by each patch of the slide, output whether a disease is developed in the patch, and/or specify a region having the disease developed in the patch. It is apparent that a neural network therefor may be trained to be implemented.

A second phase may output whether the disease is developed on the slide through the diagnosis result of the first phase. Neural networks or certain machine learning techniques may be involved for the process.

In other words, even if it is determined that some patches have a disease developed according to the diagnosis results of each patch, there may be a possibility not to be determined that the disease is developed in the corresponding biotissue over the entire slide including the patch. For example, when patches determined to have the disease developed are scattered distractingly in the slide, when the number is small, or the physical features (e.g., location, size, density, etc.) of the patches determined to have other diseases developed, such as density, may have an important meaning in determining whether a disease is developed in a corresponding slide. Therefore, the second phase may determine the diagnosis result for each patch and whether the disease is developed on the slide based on the features of patches (i.e., patches diagnosed as having a disease developed) determined based on such the diagnosis result, thereby effectively performing diagnosis with high accuracy.

On the other hand, the system 100 may include a patch level segmentation neural network for performing segmentation for each patch. There may be a problem in which the entire patch is determined to have a disease developed despite the presence of disease-free region within the patch, when whether the disease is developed is determined (i.e., classification by each patch) according to the diagnosis result of the patch unit and it is determined that the disease is developed in the patch unit. Therefore, segmentation capable of classifying disease regions within any one patch may be required to clearly grasp a tissue part diagnosed as a disease, and for this purpose, the patch level segmentation neural network may be implemented.

An example of the technical idea for such segmentation has been disclosed in Korean Patent Publication No. 10-2019-0084814 "Disease diagnosis system and method for performing segmentation using neural network and non-local block" (hereinafter referred to as 'previous application'), and the previous application may be incorporated herein as a reference.

In any case, the neural network 12-2 may be configured to determine whether the disease is developed by each patch and specify only the development region of the disease within each patch according to an embodiment.

In other words, the neural network 12-2 may determine the development region of the disease simply by patch or further specify only the development region of the disease within the patch.

In addition, the neural network 12-2 may determine not only whether a specific disease is developed, but also state information indicating a degree of progression of a specific disease (or a probability corresponding to a degree of progression).

For example, when the technical idea of the present disclosure is used to diagnose prostate cancer, Gleason pattern or Gleason score, an indicator for a degree of prostate cancer progression, may be included in the state information output by the neural network. For example, the Gleason score has a value of 2 to 5, wherein the larger the number, the more severe the development of prostate cancer. Thus, the state information may refer to a probability that the biotissue corresponding to the patch to be diagnosed corresponds to a specific value of the Gleason score (e.g., 3, 4, or 5).

In other words, the neural network 12-2 may determine not only whether the disease is developed for each patch, but also into which class the patch is classified among a plurality of classes (e.g., Gleason score 3, 4, or 5) even when the disease is developed.

When the system 100 is implemented by being included in a predetermined server 10, the system 100 may communicate with at least one client (e.g., 20, 20-1) that is accessible to the server 10. In this case, the client (e.g., 20, 20-1) may transmit the biometric image to the system 100, and the system 100 may perform diagnosis according to the technical idea of the present disclosure for the transmitted biometric image. In addition, the diagnosis result may be transmitted to the client (e.g., 20, 20-1). In addition, the system 100 may generate and output prognosis prediction information based on the diagnosis result of the biometric image.

To this end, the system 100 may include a diagnosis system and a prognosis prediction system as shown in FIG. 5.

The diagnosis system may be configured to receive a biometric image as described above and perform diagnosis. Performing of diagnosis may refer to outputting of information that may specify a region in which the disease is developed in the biometric image, that is, a development region diagnosis result. The development region diagnosis result is sufficient as long as it is information that may specify the region having the disease developed on the biometric image. For example, the image information displayed so that a region having the disease developed on the biological image is distinguished from other regions may itself be a development region diagnosis result, and information (coordinates) on pixels included in the region having the disease developed may be a development region diagnosis result. Alternatively, the identification information of the patch in which the disease is developed may be the development region diagnosis result. Various embodiments may exist to implement the development region diagnosis results.

The prognosis prediction system may generate information used for prognosis prediction. To this end, the prognosis prediction system may receive development region diagnosis results from the diagnosis system.

The prognosis prediction system may then determine first information corresponding to a size of an entire tissue on the biometric image and second information corresponding to a size of the development region on the biometric image based on the diagnosis result. In addition, prognosis prediction information based on the determination result may be generated. The prognosis prediction information may include information on the development rate as described above, but is not limited thereto, and any information that may be derived from the development region diagnosis result and have significance in prognosis prediction may be included in the prognosis prediction information.

The first information may represent information on a size of tissues, and the first information may be defined in various ways such as length, area, and number of pixels.

In order to determine the first information, the prognosis prediction system may compute the first information corresponding to the size of tissues by classifying the stained color and background of the tissue in a predetermined manner.

The second information may be information that may represent a size of a region in which the actual disease is developed among the tissues.

In addition, when the diagnosis system is implemented to classify a developed disease into any one of a plurality of classes according to a state of the disease when the disease is developed, a second information indicating the size of the development region for each class may be computed by the prognosis prediction system. For example, in the case of prostate cancer, the second information corresponding to the Gleason score 3, the second information corresponding to the Gleason score 4, and the second information corresponding to the Gleason score 5 may be determined separately.

On the other hand, the diagnosis system may simply perform diagnosis of a disease by each patch as described above, and in this case, for a patch determined as having the disease developed, the entire patch may be treated as having the disease developed (or a disease of the corresponding class).

Even in this case, as described above in FIG. 1, it may be possible to compute much more accurate prognosis prediction information than a case that the second information is determined by a conventional pathologist.

According to an embodiment, the diagnosis system may segment the region having the disease developed among the patch for each patch. In this case, only the region having the disease developed may be classified and specified within any one patch, such that the second information may be more precise, and when the second information is determined based on the result, it is possible to compute prognosis prediction information securing higher accuracy.

The classification between the diagnosis system and the prognosis prediction system as shown in FIG. 5 above is classification of logical function, rather than that the system 100 according to the technical idea of the present disclosure is implemented in a physically classified configuration. In other words, the configurations disclosed in FIGS. 3 and 4 may implement both the diagnosis system and the prognosis prediction system.

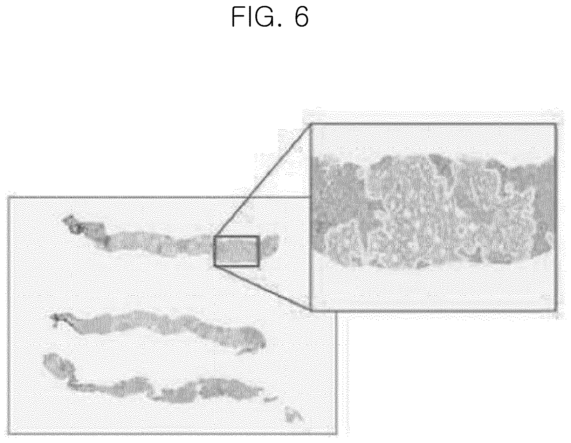

This technical idea will be illustrated with reference to FIG. 6.

FIG. 6 is a diagram illustrating a development region diagnosis result of a biometric image to describe the prognosis prediction method using a result of disease diagnosis through a neural network in accordance with an embodiment of the present disclosure.

FIG. 6 shows an example in which a tissue shown in FIG. 1 is diagnosed by the diagnosis system according to the technical idea of the present disclosure, and the development region diagnosis result is displayed on a biometric image.

In FIG. 6, a region marked in yellow is a region determined to be a predetermined first class (e.g., Gleason score 3) among the disease states, and a region marked in orange is a region determined to be a predetermined second class (e.g., Gleason score 4) among the disease states.

Comparing and referring to FIG. 6 and FIG. 1, as shown in FIG. 6, unlike a case that the pathologist marks the boundary of the region having the disease developed and considers that all the tissues in the boundary have a disease developed, according to the technical idea of the present disclosure, even within the boundary, regions with and without diseases actually developed may be classified and determined at a pixel level or a detailed region close thereto.

Therefore, in the conventional art, compared to a case that only a rough development rate is estimated, according to the technical idea of the present disclosure, relatively very accurate information on the size of the development region of the disease may be obtained.

In addition, as shown in FIG. 6, it may be possible to calculate not only the region having the disease developed, but also a state of the disease, that is, an exact region for each class. Therefore, the system 100 may set various indicators for prognosis prediction such as a ratio for the development region of the disease to the entire tissue, as well as a ratio of the development region of a first class (e.g., Gleason score 3) to the development region of the disease, a ratio of the development region of the second class to the development region of the disease (e.g., Gleason score 4), and a ratio of the development region of the second class to the development region of the first class.

The availability of such a variety of prognosis prediction information may mean that the current patient's state may be more precisely classified, and treatment or prescription may be made thereby.

On the other hand, the neural network 12-2 may perform diagnosis of a disease at a patch level as described above and determine a development region of the disease on a biometric image. Surely, in order to perform such diagnosis, a process of training the neural network may first be performed. In addition, as described above, a predetermined neural network may also be used for slide level diagnosis.

Therefore, the system 100 may be a system for performing diagnosis by receiving a neural network trained according to the technical idea of the present disclosure and a program for performing diagnosis using the neural network from the outside to perform diagnosis or a system for performing even the training of the neural network. In addition, the system 100 may be implemented as a dedicated device designed to implement the technical idea of the present disclosure, not a general-purpose data processing device, and in this case, a means for scanning a biometric image may be further provided.

The system for implementing such a technical idea may logically have the same configuration as FIG. 4.

Referring to FIG. 4, the system includes a control module 110 and a neural network module 120 in which the neural network and/or slide diagnosis engine are stored. In addition, the system 100 may further include a preprocessing module 130. According to an embodiment of the present disclosure, it is apparent that some of the above-described components may not necessarily correspond to components essential to the implementation of the present disclosure, and the system 100 may include more components according to an embodiment. For example, the system 100 may further include a communication module (not shown) for communicating with the client (e.g., 20, 20-1).

The system 100 may refer to a logical configuration having hardware resources and/or software necessary to implement the technical ideas of the present disclosure, but does not necessarily mean one physical component or one device. In other words, the system 100 may refer to a logical combination of hardware and/or software provided to implement the technical idea of the present disclosure, and if necessary, it may be implemented as a set of logical configurations to implement the technical idea of the present disclosure by being installed in a device spaced apart from each other to perform respective functions. In addition, the system 100 may refer to a set of configurations separately implemented for each function or role to implement the technical idea of the present disclosure. For example, each of the control module 110, the neural network module 120, and/or the preprocessing module 130 may be located in different physical devices or in the same physical device. In addition, according to an embodiment, the combination of software and/or hardware constituting each of the control module 110, the neural network module 120, and/or the preprocessing module 130 may also be located in each different physical device, and configurations located in each different physical device may be organically combined with each other to implement each of the modules.

In addition, the term module as used herein may refer to functional and structural combination of hardware to perform the technical ideas of the present disclosure and software to drive the hardware. For example, an average expert in the art of the present disclosure will be able to easily infer that the module may refer to a predetermined code and a logical unit of hardware resources to allow the predetermined code to be performed, and not necessarily mean physically connected code, or a type of hardware.

The control module 110 may control other configurations (e.g., the neural network module 120 and/or the preprocessing module 130) included in the system 100 to implement the technical ideas of the present disclosure.

In addition, the control module 110 may receive a biometric image and perform diagnosis according to the technical idea of the present disclosure using the neural network module 120 and/or slide diagnosis engine stored for the received biometric image. In addition, the development region diagnosis results may be output. In addition, the control module 110 may generate prognosis prediction information.

The control module 110 may receive input data, that is, input for each patch, to the patch level neural network, that is, the trained neural network stored in the neural network module 120. In addition, computing operations defined by the neural network may be performed to output output data, that is, a feature value corresponding to a probability of disease development corresponding to the patch. In addition, according to an embodiment, a region having a disease developed among the patchs may be specified. In addition, according to an embodiment, for slide level diagnosis as described below, the patch may output whether the disease is developed depending on whether the feature value is a predetermined threshold value.

The neural network module 120 may include a patch diagnosis engine for performing patch level diagnosis and a slide diagnosis engine for performing slide level diagnosis.

The patch level diagnosis engine may be implemented through a deep learning-based neural network according to the technical idea of the present disclosure as described above. The deep learning-based neural network may be used for the slide diagnosis engine, or a predetermined machine learning (e.g., XGBoost) engine, not the neural network, may be used.

The neural network may refer to a set of information expressing a series of designs defining a neural network. In the present specification, the neural network may be a convolution neural network.

As is well known, the convolution neural network may include an input layer, a plurality of hidden layers, and an output layer. Each of the plurality of hidden layers may include a convolution layer and a pooling layer (or a subsampling layer). In addition, a convolution neural network may be designed to include a normalization layer, such as a batch normalization (BN) layer.

The convolution neural network may be defined by functions, filters, strides, and weight factors to define each of these layers. In addition, the output layer may be defined as a fully connected feedforward layer.

The specification on the design of each layer that constitutes the convolution neural network is widely known. For example, known functions may be used for the number of layers to be included in the plurality of layers, the convolution function, the pooling function, and the activation function for defining the plurality of layers, and separately defined functions may be used to implement the technical idea of the present disclosure.

An example of a convolution function includes a discrete convolution sum. As an example of a pooling function, a max pooling and average pooling may be used. An example of activation functions may include sigmoid, tangent hyperbolic (tanh), rectified linear units (ReLU), Swish, and exponential linear unit (ELU).

As mentioned above, the neural network performing patch level diagnosis may be a patch level segmentation neural network that not only determines whether a disease exists in the patch but also performs segmentation to specify the development region of a disease among the patches.

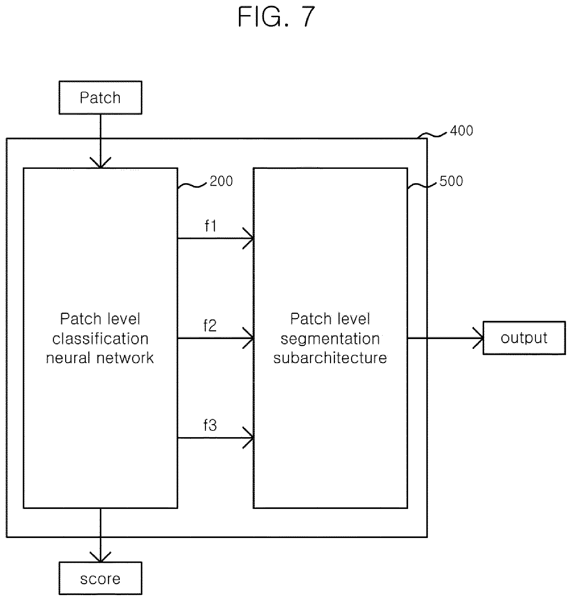

The patch level segmentation neural network according to an embodiment of the present disclosure may be implemented in a form in which a separate architecture for segmentation is combined based on a neural network ('patch level classification neural network' to be described later) that performs classification for determining whether a disease exists in the patch. The structure of such the patch level segmentation neural network is shown in FIG. 7. The patch level segmentation neural network shown in FIG. 7 is disclosed in detail in the previous application, so brief description thereof is as follows.

As shown in FIG. 7, the patch level segmentation neural network 400 according to an embodiment of the present disclosure may include a patch level classification neural network 200 and a patch level segmentation architecture 500.

The patch level classification neural network 200 may receive a patch that is a part of a divided slide as an input layer and output a patch level classification result (e.g., a score as shown in FIG. 7) regarding whether the disease exists in the patch. This is called classification, and in the process of classification, the patch level classification neural network 200 may generate a feature for an input (i.e., a patch) as an intermediate product in some hidden layers included inside. In particular, when receiving a matrix in two dimensions or more such as an image, the generated feature is in the form of a two-dimensional matrix, such that the term feature map is sometimes used. On the other hand, hereinafter, a layer that generates a feature map among hidden layers included in the patch level classification neural network 200 will be called a feature map extraction layer.

On the other hand, the patch level segmentation architecture 500 may receive feature maps (e.g., f1, f2, f3 shown in FIG. 4 of the previous application) generated from each of the two or more feature map extraction layers among the hidden layers included in the patch level classification neural network 200 and output by specifying a region where a disease exists in the patch.

Though FIG. 7 shows an example in which the patch level classification neural network 200 generates three feature maps (low-level feature map f1, mid-level feature map f2, and high-level feature map f3) in the process of performing classification, it is apparent that more or fewer feature maps may be generated according to an embodiment.

On the other hand, according to an embodiment of the present disclosure, the patch level classification neural network 200 performing patch level classification may use a known DenseNet, but is not limited thereto. In addition, various neural networks may be utilized, and in any case, the patch level classification neural network 200 may be defined to receive a specific patch as an input and output a feature value corresponding to a disease development probability of the specific patch.

For the patch level segmentation architecture 500 as described above, feature maps (f1, f2, f3) may be generated by each feature extraction layer in the classification process performed in the patch level classification network 200, and based on this, it is possible to specify the region where the disease exists in the patch. In addition, non-local correlation may be used for this purpose, as disclosed in the previous application.

As described above, when the region having the disease developed is specified by the neural network module 120, that is, the development region diagnosis result is generated, the control module 110 may generate prognosis prediction information.

The control module 110 may determine the first information corresponding to the size of the entire tissue on the biometric image subjected to diagnosis as described above. For example, the first information may be determined based on the number of pixels and/or the number of patches.

In addition, based on the diagnosis result, second information corresponding to the size of the development region on the biometric image may be determined. For example, the second information may be determined based on the number of pixels and/or patches determined to have the disease developed.

The control module 110 may then generate prognosis prediction information based on the first information and the second information.

In addition, when the neural network module 120 classifies the disease into a plurality of classes while determining whether the disease is developed by pixel unit and/or patch, the control 110 may determine second information corresponding to the size of the development region on the biometric image by each class. In addition, in this case, it is apparent that the development rate corresponding to the size of the disease development region for each class compared to the size of the entire tissue may be included in the prognosis prediction information.

The preprocessing module 130 may be configured to perform preprocessing of a biometric image necessary before performing diagnosis using a neural network. For example, the preprocessing of the biometric image may include patching of the biometric image into patches in a predefined size and calculate gray values of pixels for each patch as described above. In addition, an average expert in the art of the present disclosure will be able to easily infer that appropriate image processing may be performed in a way suitable for the neural network as needed.

On the other hand, according to an embodiment, the system 100 may include a processor and a memory configured to store a program executed by the processor. The processor may include a single-core CPU or a multi-core CPU. The memory may include a high-speed random access memory and include a non-volatile memory such as one or more magnetic disk storage devices, flash memory devices, or other non-volatile solid-state memory devices. Access to memory by the processor and other components may be controlled by a memory controller.

On the other hand, the method according to an embodiment of the present disclosure may be implemented in the form of a computer-readable program command and stored on a non-transitory computer-readable recording medium, and a control program and a target program according to an embodiment of the present disclosure may also be stored in a non-transitory computer-readable recording medium. A non-transitory computer-readable recording medium includes all types of recording devices in which data that may be read by a computer system is stored.

Program commands recorded on a recording medium may be specifically designed and configured for the present disclosure or may be known and available to those skilled in the software field.

Examples of a computer-readable recording medium include magnetic media such as hard disks, floppy disks, and magnetic tapes, optical media such as CD-ROMs and DVDs, magneto-optical media such as floptical disks, and hardware devices specifically configured to store and execute program commands such as ROMs, RAM, and flash memory. In addition, the computer-readable recording medium is distributed in computer systems connected through a network, so that computer-readable codes may be stored and executed in a distributed manner.

Examples of program commands include machine codes such as those generated by compilers, as well as devices that electronically process information using interpreters, such as high-level language code that may be executed by a computer.

The above-described hardware device may be configured to operate as one or more software modules to perform the operation of the present disclosure, and vice versa.

The foregoing description of the present disclosure is for illustrative purposes only, and a person skilled in the art to which the present disclosure pertains will be able to understand that it may be easily transformed into other concrete forms without changing the technical idea or essential features of the present disclosure. Therefore, the embodiments described above should be understood as exemplary and not limited in all respects. For example, each component described as a single type may be implemented in a distributed form, and likewise components described as a distributed form may be implemented in a combined form.

The scope of the present disclosure is indicated by the claims to be described later rather than by the above detailed description, and the meaning and scope of the claims and all modifications or modified forms derived from the concept of equivalence thereof should be construed as included in the scope of the present disclosure.

### Industrial Applicability

The present disclosure may be used for a prognosis prediction method using a result of disease diagnosis through a neural network and a system therefor.

## Claims

1. A prognosis prediction method using a result of disease diagnosis through a neural network, which is implemented by a system comprising a processor and a storage device configured to store a neural network, the prognosis prediction method comprising:
receiving, by the system, a biometric image;
generating for the received biometric image, by the system, a development region diagnosis result in which a development region having a disease developed is determined in the biometric image; and
determining, by the system, first information corresponding to a size of an entire tissue on the biometric image and second information corresponding to a size of the development region on the biometric image based on the diagnosis result and generating prognosis prediction information based on a result of the determination,
wherein the generating for the received biometric image, by the system, of the development region diagnosis result in which the development region having the disease developed is determined in the biometric image comprises
generating the development region diagnosis result based on a result of determining whether the disease is developed for each of predetermined patches obtained by dividing the biometric image into a predetermined size or generating the development region diagnosis result based on a result of segmentation of a region having the disease developed among the patches for each of the patches.

2. The method of claim 1, wherein the generating for the received biometric image, by the system, of the development region diagnosis result in which the development region having the disease developed is determined in the biometric image comprises
generating, when the disease is developed and a state of the developed disease is classified into a plurality of classes, by the system, the development region diagnosis result for each class, and
wherein the determining, by the system, of the first information corresponding to the size of the entire tissue on the biometric image and the second information corresponding to the size of the development region on the biometric image based on the diagnosis result and generating prognosis prediction information based on the result of the determination comprises
determining, by the system, the second information corresponding to the size of the development region on the biometric image for each class based on the development region diagnosis result.

3. The method of claim 1, wherein the prognosis prediction information comprises information on a development rate corresponding to the size of the development region relative to the size of the entire tissue.

4. A computer program which is installed in a data processing device and recorded on a non-transitory medium for performing the method according to any one of claims 1 or 3.

5. A system for prognosis prediction using a result of disease diagnosis through a neural network, which comprises a processor and a storage device configured to store a neural network, the system comprising:
a processor; and
a memory configured to store a program driven by the processor and a neural network,
wherein the processor is configured to drive the program to receive a biometric image, generate a development region diagnosis result in which a development region having a disease developed is determined in the biometric image for the biometric image, determine first information corresponding to a size of an entire tissue on the biometric image and second information corresponding to a size of a development region on the biometric image based on the diagnosis result, and generate prognosis prediction information based on a result of the determination, and
wherein the processor is configured to drive the program to generate the development region diagnosis result based on a result of determining whether the disease is developed for each of predetermined patches obtained by dividing the biometric image into a predetermined size or generate the development region diagnosis result based on a result of segmentation of a region having the disease developed among the patches for each of the patches.

6. The system of claim 5, wherein the processor is configured to drive the program to:
generate, when the disease is developed and a state of the developed disease is classified into a plurality of classes, the development region diagnosis result for each class; and
determine the second information corresponding to the size of the development region on the biometric image for each class based on the development region diagnosis result.
